# EUROPÄISCHE PATENTANMELDUNG

(11) **EP 3 788 984 A1**
(43) Veröffentlichungstag der Anmeldung: **10.03.2021**
(21) Anmeldenummer: 19195172.2
(22) Anmeldetag: 03.09.2019
(51) Int. Cl.: A61C 9/00, A61C 19/04, A61B 5/00

(54) **VERFAHREN ZUM BETREIBEN EINES INTRAORALSCANSYSTEMS ZUM BESTIMMEN EINES PLAQUE-INDEXES**

(71) Anmelder: ZACK Zahnarztpraxis Christina Krause, 22547 Hamburg (DE)
(72) Erfinder: KRAUSE, Christina, 22589 Hamburg (DE)
(74) Vertreter: Seemann & Partner Patentanwälte mbB

(57) **Zusammenfassung**

Die Erfindung betrifft ein Verfahren zum Betreiben eines Intraoralscansystems, umfassend einen Intraoralscanner und eine Auswertevorrichtung, die ausgebildet ist, die durch den Intraoralscanner aufgenommenen Bilder zu verarbeiten. Das erfindungsgemäße Verfahren zeichnet sich durch die folgenden Verfahrensschritte aus:

- Zurverfügungstellung eines durch den Intraoralscanner erzeugten ersten Bildes (Fig. 3b), wobei auf dem ersten Bild (Fig. 3b) wenigstens ein erster Zahn (12) abgebildet ist, der keine Färbung durch ein Zahnbelagfärbemittel aufweist,

- Zurverfügungstellung eines durch den Intraoralscanner erzeugten zweiten Bildes (Fig. 3a), wobei auf dem zweiten Bild (Fig. 3a) wenigstens der erste Zahn (12) abgebildet ist, der eine Färbung (13) durch ein Zahnbelagfärbemittel aufweist,

- Vergleich des ersten Bildes (Fig. 3b) mit dem zweiten Bild (Fig. 3a) zumindest in einem Bereich, in dem der erste Zahn (12) wenigstens teilweise abgebildet ist, und

- Bestimmen eines Zahnbelagindexes anhand des Vergleichs.

## Beschreibung

Die Erfindung betrifft ein Verfahren zum Betreiben eines Intraoralscansystems, umfassend einen Intraoralscanner und eine Auswertevorrichtung, die ausgebildet ist, die durch den Intraoralscanner aufgenommenen Bilder zu verarbeiten. Die Erfindung betrifft ferner ein Computerprogrammprodukt und die Verwendung eines Intraoralscansystems.

Intraoralscansysteme werden heutzutage verwendet, um im Mund Zähne zu scannen und vorzugsweise dreidimensional zu erfassen, um so Zahnersatz auf einfache Weise anpassen und herstellen zu können. Es wird hierzu auf die US 2016/0256035 A1 verwiesen.

Zur Kontrolle der Zahnreinigung von Personen werden heutzutage durch geschultes Personal Zahnbelagindices bzw. Plaque-Indices erstellt. Übliche Plaque-Indices sind beispielsweise die nach Silness & Löe, Quigley und Hein modifiziert nach Turesky und der Rustogi et al Modified Navy Plaque Index. Um einen Plaque-Index festzulegen, werden die Zähne mit einer Färbelösung, also einem Zahnbelagfärbemittel, wie ein Red-Cote-Liquid oder eine Färbelösung Mira2Ton, versehen. Es kann auch der Farbstoff Erytrosin verwendet werden. Der Plaque-Index nach Quigley und Hein sieht beispielsweise sechs Bewertungsgrade vor, nämlich Bewertungsgrad 0 = keine Plaque, Bewertungsgrad 1 = vereinzelte Plaque-punkte, Bewertungsgrad 2 = deutliche Plaque-Linien am Zahnfleischsaum, Bewertungsgrad 3 = Zahnbelege bedecken das zervikale Drittel der Zahnoberfläche, Bewertungsgrad 4 = Zahnbelege bedecken bis zu zwei Drittel der Zahnoberfläche und Bewertungsgrad 5 = Zahnbelege bedecken mehr als zwei Drittel der Zahnoberfläche. Diese Bewertungen werden von geschultem Personal mittels Sichtkontrolle durchgeführt und sind schon aus diesem Grund mit Unsicherheit behaftet. Wird die Plaque-Index-Bestimmung routinemäßig während der Zahnkontrolle erstellt, sind die Ergebnisse durch den Bewerter beeinflusst. Damit sind die Ergebnisse besonders über einen langen Zeitraum nicht exakt vergleichbar.

Es ist Aufgabe der vorliegenden Erfindung, die Zahnbelagindexbestimmung bzw. die Bestimmung der Menge des Zahnbelags verlässlicher und genauer zu machen.

Gelöst wird diese Aufgabe durch ein Verfahren zum Betreiben eines Intraoralscansystems, umfassend einen Intraoralscanner und eine Auswertevorrichtung, die ausgebildet ist, die durch den Intraoralscanner aufgenommenen Bilder zu verarbeiten, mit den folgenden Verfahrensschritten:
- Zurverfügungstellung eines durch den Intraoralscanner erzeugten ersten Bildes, wobei auf dem ersten Bild wenigstens eine vestibuläre und/oder orale Fläche eines ersten Zahns abgebildet ist, der keine Färbung durch ein Zahnbelagfärbemittel aufweist,
- Zurverfügungstellung eines durch den Intraoralscanner erzeugten zweiten Bildes, wobei auf dem zweiten Bild wenigstens eine vestibuläre und/oder orale Fläche des ersten Zahns abgebildet ist, der eine Färbung durch ein Zahnbelagfärbemittel aufweist,
- Vergleich des ersten Bildes mit dem zweiten Bild zumindest in einem Bereich, in dem der erste Zahn wenigstens teilweise abgebildet ist, und
- Bestimmen eines Zahnbelagindexes anhand des Vergleichs.

Überraschenderweise wurde festgestellt, dass bei der Durchführung des erfindungsgemäßen Verfahrens sehr genau und verlässlich eine Plaque-Index-Bestimmung möglich ist.

Vorzugsweise wird auf dem ersten Bild und dem zweiten Bild der erste Zahn im Wesentlichen von einer Seite vollständig abgebildet. Ferner bevorzugterweise werden mehrere Zähne von einer Seite abgebildet, so dass eine Mittelung des Plaque-Index über mehrere Zähne durchgeführt werden kann.

Vorzugsweise wird auf dem ersten Bild und dem zweiten Bild der erste Zahn im Wesentlichen sowohl von der vestibulären als auch von der oralen Fläche abgebildet. Ferner bevorzugterweise werden mehrere Zähne von den beiden Seiten abgebildet, so dass vorzugsweise der von jedem Zahn bestimmte Zahnbelagindex verwendet wird, um einen Gesamtzahnbelagindex zu erstellen. Hierbei können die jeweiligen Zahnbelagindices der jeweiligen Zähne gemittelt werden, beispielsweise durch einen arithmetischen Mittelwert oder einen geometrischen Mittelwert, es kann auch ein quadratischer Mittelwert Verwendung finden. Zudem kann als ein Bewertungskriterium für den Zahnbelagindex die Anzahl der bewerteten Zähne bzw. die Anzahl der bewerteten Abbildungen von Zähnen berücksichtigt werden. Insbesondere kann bei einem Zahnbelagindex bzw. Gesamtzahnbelagindex mehrerer Zähne die Anzahl der Zähne, die bewertet wurden, hinzugefügt werden bzw. mit gespeichert werden. Als weiteres Bewertungskriterium kann auch eine Standardabweichung Berücksichtigung finden. Sind beispielsweise einige wenige Zähne mit sehr wenig Zahnbelag bis gar keinem Zahnbelag (also einem niedrigen Zahnbelagindex) versehen und andere Zähne mit einem hohen Zahnbelagindex, kann so auch eine gute Bewertungsmöglichkeit des Gesamtzahnbelagindexes von mehreren Zähnen möglich sein.

Im Rahmen der vorliegenden Anmeldung bzw. des vorliegenden Patentes ist unter einem Plaque-Index insbesondere auch ein Zahnbelagindex zu verstehen.

Bei dem ersten Bild und dem zweiten Bild kann es sich um eine zweidimensionale Abbildung wenigstens eines Zahns handeln. Es können allerdings bevorzugt auch dreidimensionale Bilder vorgesehen sein, die miteinander verglichen werden.

Vorzugsweise wird in Bezug auf das erste Bild und/oder das zweite Bild eine Zahnnummernzugehörigkeit beispielsweise nach FDI oder einem amerikanischen Zahnschema festgelegt und dem ersten Bild und/oder dem zweiten Bild zugeordnet. Die Zahnnummernzugehörigkeit überträgt sich vorzugsweise auf jedes weitere erste und/oder zweite Bild desselben Patienten der jeweils zu dem jeweiligen Zahn gehörenden Zahnnummernzugehörigkeit. Vorzugsweise können Zähne im ersten Bild von der Bewertung für den Zahnbelagindex bzw. die Bestimmung des Zahnbelagindexes ausgeschlossen werden, beispielsweise dann, wenn dieser spezielle Zahn erhebliche Defekte aufweist. Dieser Ausschluss von Zähnen kann sich auf jedes zweite Bild oder jedes später aufgenommene erste Bild des gleichen Patienten übertragen.

Vorzugsweise wird zum Vergleichen des ersten Bildes mit dem zweiten eine Größenanpassung, eine Perspektivenanpassung und/oder eine Lageanpassung durchgeführt.

Für den Fall, dass die Größe des ersten Zahns oder des wenigstens einen ersten Zahns auf dem ersten Bild und dem zweiten Bild unterschiedlich ist, beispielsweise deswegen, weil der Intraoralscanner in einem anderen Abstand zum ersten Zahn beim Scannen des ersten Zahns angeordnet ist, findet eine entsprechende Größenanpassung des ersten und des zweiten Bildes statt, um das Abbild des ersten Zahns beim ersten Bild und beim zweiten Bild gleich groß zu haben. Entsprechend kann eine Perspektivenanpassung vorgenommen werden, wenn sich die Perspektive des Intraoralscanners zu dem ersten Zahn zwischen dem ersten Bild und dem zweiten Bild unterscheidet. Hierzu wird beispielsweise das Verhältnis der Breite zur Höhe des abgebildeten Zahns angepasst. Entsprechend kann auch eine Lageanpassung durchgeführt werden, wenn nämlich auf dem ersten Bild der erste Zahn versetzt zu der Lage des ersten Zahns auf dem zweiten Bild ist. Die Lageanpassung kann beispielsweise durch einfaches Verschieben der Bilder bzw. der Bildpunkte zueinander durchgeführt werden.

Vorzugsweise werden zum Vergleichen des ersten Bildes mit dem zweiten Bild die Bilder wenigstens im Bereich des ersten Zahns registriert. Unter Registrieren wird im Rahmen der Erfindung ein Übereinanderlegen der Bilder verstanden, so dass die Kanten bzw. Ränder der Abbildungen des ersten Zahns zumindest im Wesentlichen aufeinanderliegen bzw. übereinanderliegen.

Vorzugsweise sieht der Vergleich des ersten Bildes mit dem zweiten Bild vor, die Helligkeit der Bilder wenigstens in einem ersten Abschnitt des ersten Zahns zu vergleichen. Bei dem Vergleich der Helligkeit der Bilder kann ein Pixelvergleich der jeweiligen Bilder, die übereinandergelegt sind, vorgenommen werden. Es können allerdings auch Mittelwerte der Helligkeiten mehrerer Pixel, wie zwei Pixel, drei Pixel, vier Pixel oder eine andere Anzahl von Pixeln, die nebeneinanderliegen, vorgenommen werden. Beispielsweise kann eine Pixelfläche von beispielsweise 3 x 3 Pixeln des ersten Bildes mit einer gleich großen Pixelfläche des zweiten Bildes, die an der gleichen Stelle des Zahns liegen, als jeweiliger Helligkeitsmittelwert verglichen werden. Wenn beispielsweise zur Abbildung des Zahns 900 Pixel Verwendung finden, können so 100 gemittelte Helligkeitswerte miteinander verglichen werden. Um das Verfahren zu beschleunigen, können entsprechende Vergleiche in einer vertikalen Linie von dem Übergang der Gingiva zur Zahnoberfläche begonnen werden und für den Fall, dass zur Zahnoberkante eines Unterkieferzahns oder zur Zahnunterkante eines Oberkieferzahns kein wesentlicher Helligkeitsunterschied der beiden Bilder vorhanden ist, kann der Vergleich beendet werden. Wenn beispielsweise ein Drittel der Zahnoberfläche durch das Zahnbelagfärbemittel eingefärbt ist, ist es ab Vergleichen von dem einen Drittel des Zahns nicht mehr notwendig, saubere Zahnoberflächen miteinander zu vergleichen. Zumindest die zweite Hälfte des Zahns, bei dem ja üblicherweise, wenn nur ein Drittel des Zahns mit Zahnbelag versehen ist, keine Einfärbung mehr vorhanden ist, muss dann nicht mehr verglichen werden. Es ist also möglich, ein Abbruchkriterium für den Vergleich der Bilder vorzusehen.

Vorzugsweise sieht der Vergleich des ersten Bildes mit dem zweiten Bild vor, die Farbwerte der Bilder in wenigstens einem ersten Abschnitt des ersten Zahns zu vergleichen. Die Farbwerte können durch RGB-Farbsensoren oder CYMK-Farbsensoren des Intraoralscanners erfasst werden. Der Vergleich der Farbwerte ermöglicht eine sehr genaue Bestimmung des Plaque-Indexes. Dies liegt vor allen Dingen daran, dass beispielsweise der Rotwert eines natürlichen Zahns deutlich geringer als der Rotwert des mit dem Zahnbelagfärbemittel eingefärbten Zahnbelags ist. So kann beispielsweise der Prozentsatz des Rotwertes bei einem RGB-Farbsensor erfasst werden und ab einem gewissen prozentualen Unterschied der Rotfarbwerte des mit Färbemittel eingefärbten Zahns zu dem nicht eingefärbten Zahn bestimmt werden.

Vorzugsweise wird zum Vergleich des ersten Bildes mit dem zweiten Bild die Helligkeit und/oder der Farbwert der Bilder wenigstens in dem ersten Abschnitt des ersten Zahns durch Subtraktion oder Division der Helligkeitswerte und/oder der Farbwerte vorgenommen. Hierbei kann über vorgebbare Abschnitte des Zahns bzw. der Zahnoberfläche der Farbwert und/oder die Helligkeit integriert werden oder hiervon ein Mittelwert gebildet werden und dann ein Vergleich über Subtraktion oder Division der integrierten Abschnitte oder des Mittelwertes oder einzelner Bildpunkte vorgenommen werden. Vorzugsweise geschieht das Verfahren im Bereich des Übergangs von der Gingiva zur Zahnoberfläche mit kleineren Abschnitten oder einzelnen Bildpunkten und weiter entfernt davon mit größeren Abschnitten, da so das Verfahren schneller durchgeführt werden kann und weniger Rechenleistung benötigt. Unter Größe eines Abschnitts kann im Rahmen der Erfindung auch die Größe einer Pixelfläche mehrerer Pixel verstanden werden.

Das erste Bild, das von dem Intraoralscanner erstellt wurde und das wenigstens einen ersten Zahn abbildet, der keine Färbung durch ein Zahnbelagfärbemittel aufweist, kann durch den Intraoralscanner zu einem Zeitpunkt erstellt werden, nachdem das zweite Bild von dem Intraoralscanner erzeugt wurde, wobei das zweite Bild den wenigstens einen ersten Zahn abbildet, der eine Färbung durch ein Zahnbelagfärbemittel aufweist. Ein Patient kann somit mit Zahnbelag in eine Zahnarztpraxis kommen und dort ein Zahnfärbemittel verabreicht bekommen, woraufhin dann mit dem Intraoralscanner ein zweites Bild wenigstens eines ersten Zahns erstellt wird. Daraufhin kann eine Säuberung der Zähne stattfinden und ein erstes Bild des wenigstens einen ersten Zahns erstellt werden. Das erste Bild des wenigstens einen ersten Zahns kann allerdings auch einige Tage oder Wochen vorher, also eine deutlich größere Zeit vorher, schon erstellt worden sein und aus dem Speicher der Zahnarztpraxis bzw. aus dem Speicher des Intraoralscansystems ausgelesen werden.

Es besteht auch die Möglichkeit, dass ein neues erstes Bild von dem wenigstens einen ersten Zahn aufgenommen wird und dann als erstes Bild verwendet wird. Dies kann beispielsweise dann erforderlich sein, wenn es eine Veränderung im Zahnstatus gegeben hat, beispielweise bei einem Zahnwechsel bei Kindern oder dem Vorsehen einer Füllung.

Vorzugsweise wird auf dem ersten Bild eine Zahnunregelmäßigkeit, wie beispielsweise Zahnstein oder Füllungsflächen, erkannt, und von der Auswertung für den Zahnbelagindex wird der Bildbereich oder Bildabschnitt, der die Zahnunregelmäßigkeit aufweist, ausgeschlossen.

Vorzugsweise wird ein Gitternetz über das erste Bild und/oder das zweite Bild gelegt, wobei insbesondere der Abstand der vertikalen Gitterlinien an die Breite des jeweiligen Zahns anpassbar oder angepasst ist. Wenn beispielsweise zwei vertikale Gitterlinien für den ersten Zahn Verwendung finden, sind diese an die Breite des Zahns angepasst. Bei mehreren Gitterlinien liegen die weiteren Gitterlinien zwischen diesen beiden Gitterlinien. Die Gitterlinien können auch von der Position her von einem Benutzer des Intraoralscansystems verschoben werden, falls die Ränder der Zähne bzw. des ersten Zahns nicht exakt automatisch bestimmt worden sind. Zusätzlich können auch horizontale Gitterlinien vorgesehen sein.

Vorzugsweise sind die Gitterlinien gerade Linien. Diese können allerdings auch an den Verlauf der Ränder der Zahnoberfläche oder aufgrund eines vorgegebenen Plaque-Indexes angepasst, d.h. gekrümmt ausgebildet sein.

Vorzugsweise ist das Gitternetz an einen vorgebbaren Plaque-Index anpassbar oder angepasst. Bei dem Plaque-Index nach Quigley und Hein wäre es beispielsweise sinnvoll, horizontale Gitterlinien bei ein Drittel und zwei Drittel der Zahnoberfläche zu legen. Bei dem Rustogi et al modifizierten Navy-Plaque-Index wird die Zahnoberfläche mittels der Gitterlinien in neun Bewertungsbereiche unterteilt.

Vorzugsweise ist eine untere Gitterlinie bei einem Unterkieferzahn und/oder eine obere Gitterlinie bei einem Oberkieferzahn an den Rand eines Farbumschlags von der Gingiva auf den Zahn gelegt. Auch bei den vertikalen Gitterlinien können mehr Gitterlinien vorgesehen sein, um eine feinmaschigere Analyse des Zahnbelags und damit eine feinmaschigere Bestimmung des Plaque-Indexes zu ermöglichen.

Vorzugsweise wird ermittelt, wie viele Maschen des Gitternetzes des ersten Zahns mit einer Färbung durch ein Zahnbelagfärbemittel versehen sind und anhand dessen ein Plaque-Index bestimmt. Vorzugsweise ist vorgebbar oder definierbar, wieviel Prozent der Pixel oder Pixelflächen einen Farbumschlag aufzuweisen haben, um diese für die gesamte Masche als relevante Farbveränderung zu definieren. Die Maschen sind hierbei die durch die Gitterlinien umschlossenen Flächen oder Abschnitte auf der Zahnoberfläche.

Vorzugsweise wird eine Dicke des Zahnbelags, insbesondere in den Zahnzwischenräumen, bestimmt. Über einen Intraoral-Scanner ist es möglich, Volumina von Zähnen zu bestimmen. Um die Dicke des Zahnbelags, insbesondere in den Zahnzwischenräumen, zu bestimmen, ist es bevorzugt, eine Volumendifferenz des durch den Oral-Scanner erzeugten ersten Bildes eines ersten Zahnes und eines zweiten Bildes des ersten Zahnes durchzuführen. Die Volumendifferenz ergibt dann die Dicke des Zahnbelags, insbesondere auch die Dicke des Zahnbelags in den Zahnzwischenräumen. Um die Dicke des Zahnbelags in den Zahnzwischenräumen alleine zu bestimmen, kann die Volumendifferenz bestimmt werden und/oder eine Differenz der Breite der Abbildung des ersten und des zweiten Zahns aus oraler und/oder vestibulärer Sicht.

Vorzugsweise ist ein Computerprogrammprodukt vorgesehen, das direkt in den internen Speicher eines digitalen Computers geladen werden kann oder geladen wird und Softwarecodeabschnitte umfasst, mit denen die vorbeschriebenen erfindungsgemäßen und bevorzugten Schritte ausgeführt werden, wenn das Computerprogrammprodukt auf einem Computer läuft.

Erfindungsgemäß wird ein Intraoralscansystem zum automatischen Bestimmen eines Plaque-Indexes verwendet.

Vorzugsweise geschieht die Verwendung des Intraoralscansystems zum automatischen Bestimmen eines Plaque-Indexes mittels eines Verfahrens, das als erfindungsgemäß oder bevorzugt wie vorstehend bezeichnet wurde.

Vorzugsweise findet ein Pixelvergleich der Abbilder des ersten Zahns zwischen dem ersten und dem zweiten Bild statt. Die Pixel werden hierzu beispielsweise zunächst in die Farbbestandteile aufgeteilt und die jeweiligen Pixel von den Farbwerten des ersten mit dem zweiten Bild verglichen. Es wird vorzugsweise dann die Anzahl der identischen Pixel und der Pixel, deren Farbbestandteile bis zu einem vorgebbaren Grenzwert gleich sind, durch die Anzahl der Pixel geteilt, deren Farbbestandteile ungleich bzw. deren Farbbestandteile oberhalb des Grenzwertes unterschiedlich sind. Anhand der so bestimmten Prozentangabe wird vorzugsweise ein Plaque-Index bestimmt.

Alternativ findet kein Pixel-zu-Pixel-Vergleich statt, sondern ein Vergleich eines Integralwertes von mehreren Pixeln, die zu Gruppen von Pixeln zusammengefasst werden. Es findet somit eine Art Blockbildung oder Flächenbildung mehrerer Pixel (Pixelfläche) statt, deren Farbwerte beispielsweise aufaddiert werden.

Es kann auch eine Mittelwertbildung der jeweiligen Farbwerte einzelner Abschnitte des ersten Zahns vorgenommen werden. Hierzu kann der Zahn in eine Vielzahl von Quadraten, beispielsweise durch die Gitterlinien, aufgeteilt werden, wobei die so erzielten Maschen dann quadratförmig sind und diese jeweiligen Quadrate bzw. Maschen miteinander verglichen werden. Die jeweiligen Maschen können auch, je nach Lage auf dem Zahn, gewichtet werden. Beispielsweise kann eine Masche, die dichter am Übergang vom Zahnfleisch zur Oberfläche des Zahnes liegt, höher gewichtet sein als eine Masche, die am zahnfleischentfernten Rand der Zahnoberfläche liegt.

In einer Ausführungsform der Erfindung werden anhand des durch den Intraoral-Scanner erzeugten ersten Bildes und des durch den Intraoral-Scanner erzeugten zweiten Bildes zwei verschiedene Zahnbelagindices bestimmt. Hierzu ist vorzugsweise vorgesehen, dass die Gitterlinien bzw. das Gitternetz, die einem bestimmten Zahnbelagindex zugeordnet werden, automatisch gewechselt werden. Das Wechseln des Zahnbelagindexes durch Veränderung der Position der Gitterlinien kann manuell erfolgen. Eine automatische Umstellung ist allerdings bevorzugt.

Weitere Merkmale der Erfindung werden aus der Beschreibung erfindungsgemäßer Ausführungsformen zusammen mit den Ansprüchen und den beigefügten Zeichnungen ersichtlich. Erfindungsgemäße Ausführungsformen können einzelne Merkmale oder eine Kombination mehrerer Merkmale erfüllen.

Im Rahmen der Erfindung sind Merkmale, die mit "insbesondere" oder "vorzugsweise" gekennzeichnet sind, als fakultative Merkmale zu verstehen.

Die Erfindung wird nachstehend ohne Beschränkung des allgemeinen Erfindungsgedankens anhand von Ausführungsbeispielen unter Bezugnahme auf die Zeichnungen beschrieben, wobei bezüglich aller im Text nicht näher erläuterten erfindungsgemäßen Einzelheiten ausdrücklich auf die Zeichnungen verwiesen wird. Es zeigen:
- Fig. 1: schematisch einen Teil eines Intraoralscanbildes, das zwei vollständige Zähne zeigt,
- Fig. 2: einen Ausschnitt aus dem Bild der Fig. 1 mit Gitterlinien,
- Fig. 3a: ein schematisches zweites Bild eines Zahns mit Färbung durch ein Zahnbelagfärbemittel,
- Fig. 3b: den Zahn aus Fig. 3a, allerdings ohne Färbung durch ein Zahnbelagfärbemittel,
- Fig. 4: einen weiteren Zahn in schematischer Darstellung mit Färbung durch ein Zahnbelagfärbemittel, und
- Fig. 5: schematisch eine vestibuläre Abbildung eines ersten Zahns mit Gitterlinien nach einem Rustogi et al modifizierten Navy-Plaque-Index.

In den Zeichnungen sind jeweils gleiche oder gleichartige Elemente und/oder Teile mit denselben Bezugsziffern versehen, so dass von einer erneuten Vorstellung jeweils abgesehen wird.

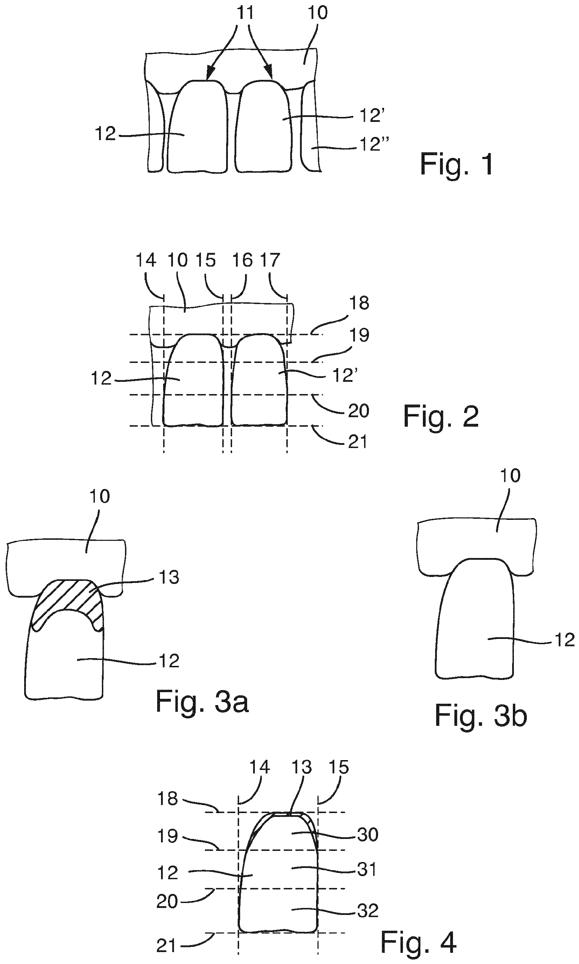

Fig. 1 zeigt einen Ausschnitt eines durch einen Intraoralscanner erzeugten Bildes von dem Inneren eines Mundraums. Es sind dort Zähne 12, 12' und 12" zu erkennen, die nach oben in einer Gingiva 10 (Zahnfleisch) liegen. Die Gingiva 10 weist zum Zahn 12, 12', 12" einen Gingiva-Rand 11 auf.

Fig. 2 zeigt einen Teil der schematischen Darstellung der Fig. 1, wobei Gitterlinien 14 bis 21 eingezeichnet sind. Die Gitterlinien 14, 15, 16 und 17 sind vertikale Gitterlinien, die den Rand der Zähne 12 und 12' zeigen. Es können auch weitere vertikale Gitterlinien vorgesehen sein, um die Oberfläche der Zähne 12 und 12' in kleinere Abschnitte bzw. Maschen aufzuteilen. Die vertikalen Gitterlinien 18 bis 21 definieren im Hinblick auf die vertikale Gitterlinie 18 den Gingiva-Rand 11 bzw. liegen an dem Gingiva-Rand 11 und definieren das Ende der Zähne 12 und 12' entfernt von dem Zahnfleisch 10. Dazwischen sind noch zwei weitere vertikale Gitterlinien 19 und 20 eingezeichnet, so dass die Zähne 12 und 12' in Drittelzähne aufgeteilt sind. Diese Aufteilung der vertikalen Gitterlinien 18 bis 21 eignet sich besonders gut für einen Zahnbelagsindex nach Quigley und Hein, da dieser bei einem Bewertungsgrad 3 eine Bedeckung durch Zahnbelag von einem Drittel der Zahnoberfläche, bei einem Bewertungsgrad von 4 eine Bedeckung bis zu zwei Drittel der Zahnoberfläche und bei einem Bewertungsgrad 5 eine Bedeckung von mehr als zwei Drittel der Zahnoberfläche vorsieht.

Fig. 3a zeigt schematisch ein Bild eines Zahns 12, der eine Zahnbelagsfärbung 13 aufweist, die einem Bewertungsgrad 3 nach Quigley und Hein entspräche.

Fig. 3b zeigt eine schematische Abbildung entsprechend Fig. 3a, allerdings ohne die Zahnbelagsfärbung 13. Zum Vergleich und zur Bestimmung des Plaque-Indexes werden die Bilder gemäß Fig. 3a und Fig. 3b miteinander verglichen.

Fig. 4 zeigt schematisch einen weiteren Zahn 12 mit einer Zahnbelagsfärbung 13, die weniger stark ausgeprägt ist als in Fig. 3a. Hier sind vertikale Gitterlinien 14 und 15 dargestellt, die die äußeren Ränder darstellen, und horizontale Linien 18, 19, 20 und 21. Durch diese Gitterlinienaufteilung ergeben sich drei Maschen 30, 31 und 32 bei dem Bild der Fig. 4 des Zahns 12. Es können auch feinere Unterteilungen vorgenommen werden, beispielsweise durch Vorsehen weiterer vertikaler oder weiterer horizontaler Gitterlinien. Hierdurch kann der Plaque-Index noch genauer bestimmt werden.

Fig. 4 zeigt beispielsweise schematisch einen Zahn mit einem Plaque-Index 2 bis 3 nach Quigley und Hein.

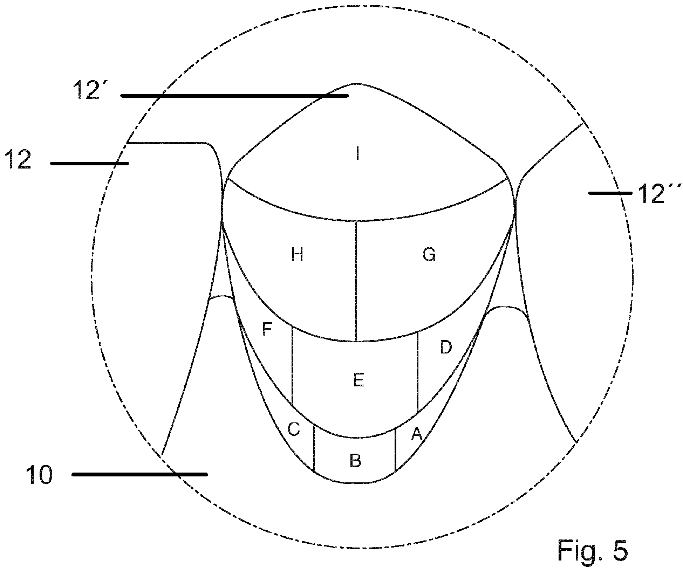

Fig. 5 zeigt schematisch eine vestibuläre Ansicht eines vollständigen Zahns sowie einen Teil der danebenliegenden Zähne, wobei der vollständige Zahn Gitterlinien aufweist, die gemäß einem Rustogi et al modifizierten Navy-Plaque-Index eingeteilt sind. Hierdurch ergeben sich neun verschiedene Zahnbereiche A bis I, die Bereiche A, B und C sind die Bereiche des gingivalen Randes. Die Bereiche G und F sind proximale Bereiche des Zahns. Das Vorsehen derartiger Gitterlinien führt zu einem sehr genauen und sehr gut vergleichbaren Plaque-Index.

Alle genannten Merkmale, auch die den Zeichnungen allein zu entnehmenden sowie auch einzelne Merkmale, die in Kombination mit anderen Merkmalen offenbart sind, werden allein und in Kombination als erfindungswesentlich angesehen. Erfindungsgemäße Ausführungsformen können durch einzelne Merkmale oder eine Kombination mehrerer Merkmale erfüllt sein.

### Bezugszeichenliste

- 10: Gingiva
- 11: Gingiva-Rand
- 12, 12', 12": Zahn
- 13: Zahnbelagsfärbung
- 14: vertikale Gitterlinie
- 15: "
- 16: "
- 17: "
- 18: horizontale Gitterlinie
- 19: "
- 20: "
- 21: "
- 30: Masche
- 31: Masche
- 32: Masche
- A - I: Zahnbereiche

## Patentansprüche

1. Verfahren zum Betreiben eines Intraoralscansystems, umfassend einen Intraoralscanner und eine Auswertevorrichtung, die ausgebildet ist, die durch den Intraoralscanner aufgenommenen Bilder zu verarbeiten, mit den folgenden Verfahrensschritten:
- Zurverfügungstellung eines durch den Intraoralscanner erzeugten ersten Bildes (Fig. 3b), wobei auf dem ersten Bild (Fig. 3b) wenigstens eine vestibuläre und/oder orale Fläche eines ersten Zahns (12) abgebildet ist, der keine Färbung durch ein Zahnbelagfärbemittel aufweist,
- Zurverfügungstellung eines durch den Intraoralscanner erzeugten zweiten Bildes (Fig. 3a), wobei auf dem zweiten Bild (Fig. 3a) wenigstens die vestibuläre und/oder orale Fläche des ersten Zahns (12) abgebildet ist, der eine Färbung (13) durch ein Zahnbelagfärbemittel aufweist,
- Vergleich des ersten Bildes (Fig. 3b) mit dem zweiten Bild (Fig. 3a) zumindest in einem Bereich, in dem der erste Zahn (12) wenigstens teilweise abgebildet ist, und
- Bestimmen eines Zahnbelagindexes anhand des Vergleichs.

2. Verfahren nach Anspruch 1, **dadurch gekennzeichnet, dass** zum Vergleichen des ersten Bildes (Fig. 3b) mit dem zweiten Bild (Fig. 3a) eine Größenanpassung, eine Perspektivenanpassung und/oder eine Lageanpassung durchgeführt wird.

3. Verfahren nach Anspruch 1 oder 2, **dadurch gekennzeichnet, dass** zum Vergleichen des ersten Bildes (Fig. 3b) mit dem zweiten Bild (Fig. 3a) die Bilder (Fig. 3b, 3a) wenigstens im Bereich des ersten Zahns (12) registriert werden.

4. Verfahren nach einem der Ansprüche 1 bis 3, **dadurch gekennzeichnet, dass** der Vergleich des ersten Bildes (Fig. 3b) mit dem zweiten Bild (Fig. 3a) vorsieht, die Helligkeit der Bilder (Fig. 3b, Fig. 3a) wenigstens in einem ersten Abschnitt des ersten Zahns zu vergleichen.

5. Verfahren nach einem der Ansprüche 1 bis 4, **dadurch gekennzeichnet, dass** der Vergleich des ersten Bildes (Fig. 3b) mit dem zweiten Bild (Fig. 3a) vorsieht, die Farbwerte der Bilder (Fig. 3b, Fig. 3a) in wenigstens einem ersten Abschnitt des ersten Zahns (12) zu vergleichen.

6. Verfahren nach Anspruch 4 oder 5, **dadurch gekennzeichnet, dass** zum Vergleich des ersten Bildes (Fig. 3b) mit dem zweiten Bild (Fig. 3a) die Helligkeit und/oder Farbwerte der Bilder (Fig. 3a, 3b) wenigstens in dem ersten Abschnitt des ersten Zahns (12) durch Subtraktion oder Division der Helligkeitswerte und/oder Farbwerte geschieht.

7. Verfahren nach einem der Ansprüche 1 bis 6, **dadurch gekennzeichnet, dass** ein Gitternetz (14-21) über das erste Bild (Fig. 3b) und/oder das zweite Bild (Fig. 3a) gelegt wird, wobei insbesondere der Abstand der vertikalen Gitterlinien (14-17) an die Breite des jeweiligen Zahns (12) anpassbar oder angepasst ist.

8. Verfahren nach Anspruch 7, **dadurch gekennzeichnet, dass** das Gitternetz (14-21) an einen vorgebbaren Plaque-Index anpassbar oder angepasst ist.

9. Verfahren nach Anspruch 7 oder 8, **dadurch gekennzeichnet, dass** eine untere Gitterlinie bei einem Unterkieferzahn und/oder eine obere Gitterlinie (18) bei einem Oberkieferzahn an den Rand eines Farbumschlags von der Gingiva (10) auf den Zahn (12) gelegt ist.

10. Verfahren nach einem der Ansprüche 7 bis 9, **dadurch gekennzeichnet, dass** ermittelt wird, wie viele Maschen (30-32) des Gitternetzes (14-21) des ersten Zahns (12) mit einer Färbung (13) durch ein Zahnbelagfärbemittel versehen sind und anhand dessen ein Plaque-Index bestimmt wird.

11. Verfahren nach einem der Ansprüche 1 bis 10, **dadurch gekennzeichnet, dass** zur Bestimmung des Plaque-Indexes von mehreren Zähnen erste Bilder und zweite Bilder zur Verfügung gestellt und verglichen werden, wobei ein Mittelwert der Plaque-Indices der jeweiligen Zähne und/oder eine Standardabweichung der Plaque-Indices der jeweiligen Zähne bestimmt wird.

12. Verfahren nach einem der Ansprüche 1 bis 11, **dadurch gekennzeichnet, dass** das erste Bild zeitlich nach dem zweiten Bild aufgenommen wird.

13. Computerprogrammprodukt, das direkt in den internen Speicher eines digitalen Computers geladen werden kann oder geladen wird und Softwarecodeabschnitte umfasst, mit denen die Schritte gemäß einem der Ansprüche 1 bis 12 ausgeführt werden, wenn das Computerprogrammprodukt auf einem Computer läuft.

14. Verwendung eines Intraoralscansystems zum automatischen Bestimmen eines Plaque-Indexes.

15. Verwendung nach Anspruch 14, **dadurch gekennzeichnet, dass** zum Bestimmen des Plaque-Indexes ein Verfahren nach einem der Ansprüche 1 bis 10 ausgeführt wird.
